# EUROPÄISCHE PATENTANMELDUNG

(11) **EP 3 845 899 A1**
(43) Veröffentlichungstag der Anmeldung: **07.07.2021**
(21) Anmeldenummer: 19212907.0
(22) Anmeldetag: 02.12.2019
(51) Int. Cl.: G01N 33/36

(54) **VERFAHREN UND VORRICHTUNG ZUR WEISSGRADMESSUNG UND/ODER ANFÄRBKONTROLLE VON FILAMENTEN**

(71) Anmelder: LENZING AKTIENGESELLSCHAFT, 4860 Lenzing (AT)
(72) Erfinder: KURZBÖCK, Erich, 4690 Schwanenstadt (AT); RAMSAUER, Christoph, 5360 St. Wolfgang (AT); UNTERBERGER, Christa, 4863 Seewalchen (AT); OSTASZEWSKI, Dominik, 4690 Rüstorf (AT); SCHREMPF, Christoph, 4701 Bad Schallerbach (AT)
(74) Vertreter: Grünecker Patent- und Rechtsanwälte PartG mbB

(57) **Zusammenfassung**

Die vorliegende Erfindung betrifft ein Verfahren und eine Vorrichtung zur Weißgradmessung und/oder Anfärbkontrolle von Filamenten.

## Beschreibung

Die vorliegende Erfindung betrifft ein Verfahren und eine Vorrichtung zur Weißgradmessung und/oder Anfärbkontrolle von Garnen und Filamenten.

### Stand der Technik

Garne und Filamente, insbesondere auf der Grundlage von Cellulose werden in großem Maßstab hergestellt und in vielen Bereichen, wie Textilindustrie aber auch in technischen Bereichen, eingesetzt. Ein Beispiel derartigen Filamente sind Filamente, die nach dem Lyocellverfahren aus einer Zusammensetzung von Cellulose in einem Lösungsmittel, üblicherweise eine Mischung aus Wasser und N-Methylmorpholin-N-oxid (NMNO), hergestellt werden. Im Hinblick auf die Qualitätskontrolle der erzeugten Filamente sind neben mechanischen Kennwerten auch der Weißgrad sowie die Anfärbbarkeit relevant.

Bei der Herstellung von Garnen, wie Stapelfasergarnen, Filamentgarnen (Mono- und Multifilamentgarne), die nachfolgend als Garne/Filamentgarne bezeichnet werden, insbesondere Lyocell-Filamentgarne, werden die erzeugten Garne/Filamentgarne nach dem Spinnverfahren und ggf. nach optionalen Nachbehandlungen auf Spulen aufgewickelt und zur weiteren Nutzung bereitgestellt. Aufgrund der hohen Produktionsgeschwindigkeiten fallen dabei in einer Lyocell-Anlage pro Tag ggf. mehrere Tausend derartiger Spulen an, die dann der Nachkontrolle gewünschter Eigenschaften zugeführt werden müssen. Dabei sollen, insbesondere im Hinblick auf die Kontrolle des Weißgrads und der Anfärbbarkeit, die erhaltenen Resultate möglichst exakt und reproduzierbar sein. Gleichzeitig sollte der Aufwand (Arbeitsaufwand ebenso wie Platzbedarf der Vorrichtungen) für diese Messungen so gering wie möglich sein und vorzugsweise ein im wesentlicher automatisierter Ablauf realisierbar sein.

Derzeit werden zur Weißgradmessung und/oder Anfärbkontrolle von Garnen/Filamentgarnen, insbesondere Lyocell-Filamentgarnen, Prüfmuster gestrickt (Strickschlauch), die dann zur Weißgradmessung und/oder Anfärbkontrolle verwendet werden. Dies sind weitgehend noch manuell durchzuführende Bestimmungen, so dass eine Streuung der Ergebnisse durch den Einflussfaktor "Mensch" unumgänglich ist. Dabei hat es sich insbesondere gezeigt, dass die Resultate dieser Kontrollen fehlerhaft sein können, beispielsweise da die gestrickten Proben bei den Messungen durch unterschiedliche mechanische Belastungen (z.B. Dehnung der Probe) zu Verfälschungen der Messwerte neigen. Auch ist bei derartigen Proben ein Abgleich gegen ein Standardmaterial schwierig. Weiterhin müssen die jeweiligen Strickköpfe an die Titer der zu prüfenden Filamente angepasst werden. Dies führt durch den dabei notwendigen Austausch der Strickköpfe zu Stillstandszeiten, eine Automatisierung oder kontinuierliche Prüfung einer Vielzahl an Proben ist ebenfalls nicht möglich. Strickmaschinen sind darüber hinaus auch vergleichsweise störanfällig, was erneut zu Stillstandszeiten führen kann. Auch ist der Platzbedarf für die Lagerung der Proben vergleichsweise hoch.

### Aufgabe der vorliegenden Erfindung

Es ist daher die der vorliegenden Erfindung zugrundeliegende Aufgabe, ein Verfahren und eine Vorrichtung anzugeben, die eine Weißgrad- und/oder Anfärbkontrolle, bevorzugt beides, bei Filamentgarnen ermöglicht, insbesondere bei Lyocell-Filamenten, die die Nachteile des Standes der Technik überwinden.

### Kurze Beschreibung der Erfindung

Die vorliegende Erfindung stellt daher ein Verfahren nach Anspruch 1 und eine Vorrichtung nach Anspruch 8 zur Verfügung. Bevorzugte Ausgestaltungen sind in den Unteransprüchen und der nachfolgenden Beschreibung angegeben.

### Kurze Beschreibung der Figur

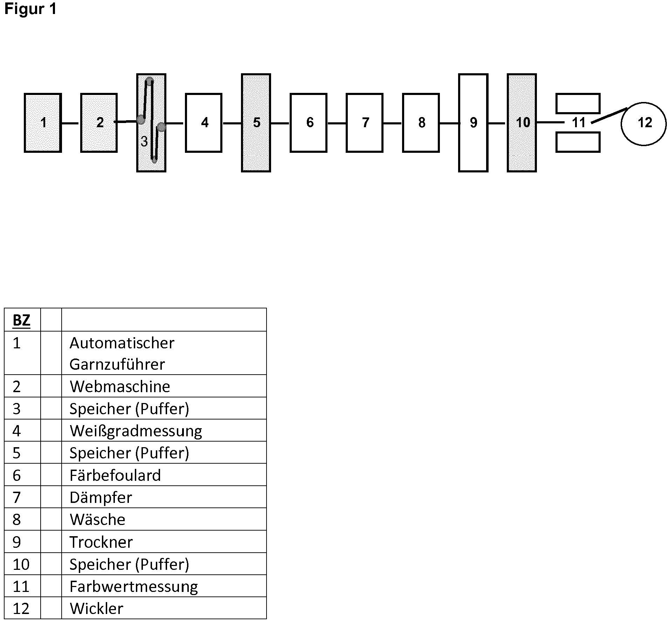

Figur 1 zeigt schematisch einen Verfahrensablauf der vorliegenden Erfindung bzw. eine schematische Darstellung einer erfindungsgemäßen Vorrichtung.

### Detaillierte Beschreibung der Erfindung

Es hat sich unerwartet gezeigt, dass durch die Verwendung von gewebten Proben anstelle von gestrickten Proben die Nachteile aus dem Stand der Technik überwunden werden können. Nachfolgend wird die Erfindung zunächst im Hinblick auf das beanspruchte Verfahren detailliert beschrieben. Es ist allerdings für den Fachmann klar, dass die folgenden Ausführungen ebenfalls im Hinblick auf die beanspruchte Vorrichtung gelten.

Im Hinblick auf die zu evaluierenden Garne/Filamentgarne ist die vorliegende Erfindung nicht auf einen Garntyp beschränkt. Es können Filamentgarne, also sowohl Mono- als auch Multifilamentgarne geprüft werden, ebenso wie andere Garnarten, wie Stapelfasergarne. Zur Vereinfachung werden diese unterschiedlichen Typen nachfolgend mit dem Begriff Garn/Filamentgarn bezeichnet.

Wie bereits ausgeführt, sind die Garne/Filamentgarne bevorzugt Lyocell-Garne/Filamentgarne. Es können aber ebenso andere Garne/Filamentgarne auf Cellulosebasis eingesetzt werden. Geeignet sind insbesondere auch cellulosische Garne/Filamentgarne, erhalten nach dem Viskoseverfahren, dem Cuproverfahren oder durch Regenration aus ionischen Flüssigkeiten.

Erfindungsgemäß wesentlich ist, dass für die Weißgrad- und/oder Anfärbkontrolle eine gewebte Probe verwendet wird. Durch den Einsatz einer gewebten Probe leidet die Kontrolle der hier relevanten Eigenschaften insbesondere nicht unter den Schwankungen und Unregelmäßigkeiten der Messungen, die durch die geringere mechanische Festigkeit der gestrickten Proben im Stand der Technik auftreten. Gleichzeitig sind die zur Herstellung der gewebten Proben einzusetzenden Vorrichtungen, bevorzugt Bandwebmaschinen, weniger störungsanfällig als Strickmaschinen, so dass eine größere Anzahl an Spulen mit Garn/Filamentgarn pro Zeiteinheit geprüft werden kann.

Bei der Herstellung der gewebten Proben, die beispielsweise die Form eines rechteckigen Gewebes erhalten, werden die zu prüfenden Garne/Filamentgarne bevorzugt als Schussfäden eingesetzt. Als Kettfäden werden bevorzugt Standardfilamentgarne eines genau definierten Titers und eines bekannten Weißgrads eigesetzt. Diese Kettfäden können Lyocel-Garne/Filamentgarne sein, jedoch ist auch der Einsatz an Kettfäden aus anderen Materialien möglich und in Ausführungsformen sogar bevorzugt. Geeignet sind beispielsweise andere Naturgarne oder Synthesegarne. Ein Beispiel eines synthetischen Fadens sind Polyesterfäden, die in gleichbleibender, standardisierter Qualität kommerziell einfach erhältlich sind. Durch den Einsatz derartiger Kettfäden kann, aufgrund der höheren Festigkeit dieser Materialien, erneut die Festigkeit der gewebten Probe erhöht werden, was den Vorteil des Einsatzes einer gewebten Probe (im Vergleich mit gestrickten Proben) weiter vergrößert. Die Kettfäden können Mono- oder Multifilamentfäden sein, geeignet sind auch alle anderen Arten an Garnen und Zwirnen. Da derartige Bandwebmaschinen Gewebe unabhängig vom Titer der Schussfäden in gleicher Qualität herstellen können (beispielsweise durch Anpassung der Schussanzahl, etwas, das automatisch bei der Herstellung der Probestücke unter Berücksichtigung des Titers des zu evaluierenden Filaments erfolgen kann), ist auch keine besondere Anpassung der Bandwebmaschine als solches an unterschiedliche Garn/Filamentgarntypen (Titer) notwendig. So können ohne großen apparativen Aufwand (keine Umbauarbeiten notwendig, anders als bei den Strickmaschinen, bei denen ein Austausch der Strickköpfe in Abhängigkeit vom Titer der zu prüfenden Filamentgarne notwendig ist) Proben ausgehend von unterschiedlichen Filamentgarntypen erzeugt werden.

Da die Einführung von Filamentgarnen in derartige Bandwebmaschinen durch den Einsatz kommerziell erhältlicher Garnzuführer möglich ist, die entweder automatisch jeweils den Anfang eines neuen Filamentgarns (typischer Weise von einer Spule) mit dem Ende des zuvor zugeführten Filamentgarns verbinden, oder den Anfang eines neuen Filamentgarns direkt durch einen Automaten an der Webnadel eingelen und anschließend fixieren, können so kontinuierlich Probenmuster (beispielsweise gewebte Proben von etwa 6 x 10 cm) von Garnen/Filamentgarnen verschiedener Spulen erzeugt werden. Durch eine entsprechende Kontrolle, beispielsweise des Pausierens der Webnadel, was Filamentgarnverbindungen zwischen den einzelnen gewebten Probestücken ergibt, kann einfach zwischen den einzelnen gewebten Proben unterschieden werden. Diese gewebten Konstruktion bildet weiterhin eine Verbindung zwischen dem jeweiligen Anfang bzw. Ende der zu prüfenden Probenmuster, so dass im Hinblick auf die noch stattfindende Kontrolle des Weißgrads und/oder der Anfärbbarkeit weiterhin eine kontinuierliche und auch automatisierte Verfahrensführung möglich bleibt.

Wie bereits ausgeführt, kann als Kettfaden ein geeignetes Garn, bevorzugt Mono- oder Multifilamentgarn eingesetzt werden. Dieses kann webvorbereitet sein, zum Beispiel durch Vorsehen einer Schlichte oder durch Präparation mit einem Öl. Geeignet sind insbesondere gedrehte, gezwirnte, interminglierte, oder geflämmte Garne/Filamentgarne. Geeignet sind insbesondere Garne/Filamentgarne all dieser Varianten mit einem Titer von 60 bis 160 dtex, bevorzugt 80 bis 120 dtex. Diese Kettfäden haben, bei gleichbleibender Bindungsart (Webbedingungen) nur einen vernachlässigbaren Einfluss auf die hier relevanten und zu bestimmenden Eigenschaften. Das zu prüfende Garn/Filamentgarn wird als Schussfaden verwendet, so dass das gewebte Probenstück eine dominierende, schussbetonte Seite aufweisen wird, an der die relevanten Messungen dann durchgeführt werden können. Durch Anpassung der Schussdichte, können auch Garne/Filamentgarne unterschiedlicher Titer zu vergleichbaren Gewebeproben verarbeitet werden. Im Allgemeinen gilt, dass bei feineren Titern die Schussdichte erhöht wird, um insbesondere ausreichend hohe Gewebefestigkeitswerte und Schussfadendichten als Messfläche sicherzustellen. Beispielhafte Kombinationen an Titer der zu evaluierenden Garne/Filamentgarne und Schussdichte sind wie folgt: (Titer Schussfaden/Schussdichte) 40dtex/26 Schuss pro cm; 500dtex/10 Schuss pro cm (bei Einsatz eines Kettbaums mit 25 Kettfäden pro cm und Atlasbindung 3/1).
Es hat sich gezeigt, dass auf diese Art und Weise Garne/Filamentgarne mit unterschiedlichen Titern (Schussfadenstärken), beispielsweise von 10 bis 500 dtex, problemlos zu Gewebeproben für die nachfolgende Weißgradbestimmung und/oder Anfärbkontrolle verarbeitet werden können.

Durch den Einsatz gewebter Proben ist es also bereits möglich mehrere der im Stand der Technik auftretenden Nachteile zu überwinden. So lassen sich Probestücke von Spulen in einer automatisierten und kontinuierlichen Weise herstellen, da durch kommerziell erhältliche Bandwebmaschinen und Garnzuführer eine automatisierte und kontinuierliche Probenherstellung realisierbar ist. Diese Anlagen arbeiten auch weniger störungsanfällig als Strickmaschinen und Stillstandszeiten aufgrund des bei der Herstellung von gestrickten Proben ggf. notwendigen Austauschs des Strickkopfes (für die Kontrolle von Spulen mit unterschiedlichen Filamentgarn-Titern) entfallen. Gleichzeitig wird durch die Verknüpfung oder die automatisierte direkte Garn-/Filamentgarnzugabe, bei den gewebten Proben eine weitere kontinuierliche Evaluierung vereinfacht, ohne dass dafür beispielsweise manuell Verknüpfungen/Verbindungen erzeugt werden müssen. Im Vergleich mit der Herstellung gestrickter Proben ist die Herstellung gewebter Proben auch schneller, was den möglichen Spulendurchsatz erhöht. Die gewebten Proben sind darüber hinaus mechanisch weniger anfällig, so dass die Messungen von Weißgrad und/oder Anfärbbarkeit weniger anfällig für Fehler/Abweichungen sind. Durch den Einsatz eines Garns/Filamentgarns mit bekannten Eigenschaften als Kettfaden, kann dieses darüber hinaus theoretisch auch als interner Standard in den Gewebeproben angesehen und entsprechend bei nachfolgenden Evaluierungen eingesetzt werden.

Die wie vorstehend beschrieben hergestellten Proben können dann anschließend, individuell und manuell, bevorzugt aber automatisiert und kontinuierlich, der weiteren Evaluierung zugeführt werden.

Eine erfindungsgemäß durchzuführende Evaluierung ist die Bestimmung des Weißgrads. Diese Bestimmung erfolgt, wie bereits vorstehend beschrieben, an der dominierenden schussbetonten Seite. Dadurch wird der Einfluss der eingesetzten Kettfäden für die Messung irrelevant. Die zur Bestimmung des Weißgrads notwendigen Bedingungen und Apparate sind dem Fachmann bekannt. Es können bekannte, kommerziell erhältliche Messapparate eingesetzt werden.

Eine weitere erfindungsgemäß durchzuführende Evaluierung ist eine Anfärbkontrolle der Garne/Filamentgarne. Dazu wird die gewebte Probe durch ein standardisiertes Verfahren angefärbt und anschließend der Farbwert bestimmt. Hierbei wird üblicherweise das Gewebe zunächst einem Farbauftrag in einem Färbefoulard unterworfen, gefolgt von Fixierung, beispielsweise in einem Dämpfer, Wäsche und Trocknung. Geeignete Farbstoffe sind insbesondere Direktfarbstoffe, wie Solophenylblau. Wesentlich im Hinblick auf die Auswertung und Vergleichbarkeit der Messergebnisse sind gleichbleibende Anfärbbedingungen, was dem Fachmann bekannt ist, ebenso wie die Bedingungen bei den einzelnen Schritten (wie Anfärbung, Fixierung, Wäsche, Trocknung). Durch das erfindungsgemäße Verfahren, das kontinuierlich gefahren werden kann, ist es möglich den Chemikalieneinsatz bei der Anfärbkontrolle, im Vergleich mit den nicht kontinuierlichen Anfärbkontrollen mit gestrickten Proben, zu minimieren.

Nach Abschluss der Färbung erfolgt die Farbwertbestimmung. Erneut erfolgt diese auf der dominierenden schussbetonten Seite des Gewebes, so dass erneut der Einfluss des Kettfadens auf das Ergebnis der Bestimmung vernachlässigbar ist. Auch hierzu sind dem Fachmann die notwendigen Bedingungen und Anlagen bekannt. Es können ebenfalls bekannte und kommerziell erhältliche Messapparate eingesetzt werden.

Erfindungsgemäß bevorzugt werden sowohl eine Bestimmung des Weißgrads als auch eine Bestimmung des Farbwerts, in dieser Reichenfolge durchgeführt. Durch die bereits vorstehend beschriebene Verbindung zwischen den einzelnen gewebten Probestücken, können diese wiederum kontinuierlich und automatisiert durch die einzelnen Schritte der Bestimmung des Weißgrads und des Farbwerts geführt werden.

Nach der Bestimmung des Farbwerts können die Proben der Lagerung zugeführt werden. Dazu können diese beispielsweise aufgewickelt werden, so dass Rollen mit den einzelnen, bevorzugt immer noch verbundenen Probestücken erhalten werden. Derartige Rollen lassen sich einfach und mit geringem Platzbedarf lagern, ein weiterer Vorteil gegenüber dem Einsatz gestrickter Proben, da hier der Platzbedarf für die Lagerung größer und auch mit höherem manuellen Aufwand verbunden ist.

Da einerseits die Herstellung der gewebten Probestücke und andererseits die Bestimmung des Weißgrads sowie die Anfärbung und Farbwertbestimmung unterschiedlich lange dauern, werden in Ausführungsformen die Probenstücke zwischen den vorstehend genannten Schritten zwischengelagert. Aufgrund der normaler Weise höheren Zeiterfordernis des Anfärbschritts, im Vergleich mit dem Weben der Probestücke sowie der Weißgradbestimmung und Farbwertmessung, sind insbesondere vor der Anfärbstufe Zwischenlagerungen der gewebten Probestücke in vielen Ausführungsformen hilfreich. Bei kontinuierlichen Verfahren kann auch eine derartige Zwischenlagerung durch Anpassung der Länge der Verbindungen (die Verknüpfung zwischen Filamentgarnende und Filamentgarnanfang zweier Filamentgarne, die beispielsweise aus nacheinander zu prüfenden Spulen stammen) zwischen den einzelnen gewebten Probestücken erfolgen. So kann ein weitestgehend automatisiertes und kontinuierliches Verfahren realisiert werden. Dabei werden die miteinander verbundenen gewebten Probestücke kontinuierlich durch geeignete Transportsysteme den einzelnen Schritten des Verfahrens zugeführt.

Unter Verweis auf Figur 1 kann also ein erfindungsgemäßes Verfahren die folgenden Schritte umfassen:
1.) Die aus der Filamentherstellung stammenden Spulen mit Filamentgarn (also beispielsweise aus der Spulmaschine) werden nacheinander in den automatischen Garnzuführer (1) eingebracht. Der Anfang eines neuen Filament (von einer neuen Spule) kann von diesem in die Webmaschine eingebracht und dabei automatisch mit dem Ende des Filaments der vorherigen Spule verknüpft werden. Alternativ kann jedes Garn/Filamentgarn auch frisch direkt eingelegt werden (siehe auch [0012]).
2.) Von der Spule wird eine ausreichende Länge an Filamentgarn abgezogen und in einer Webmaschine (2), beispielsweise einer Bandwebmaschine, zu einem Probenstück gewebt. Das zu prüfende Filamentgarn wird dabei als Schussfaden eingesetzt.
3.) Nachdem ein ausreichend großes Probestück gewebt wurde (beispielsweise mit einer Breite von etwa 10 cm und einer Länge von 6 cm) und ein ausreichend langes Endstück des Filamentgarns zusätzlich abgezogen wurde (für die mögliche Verknüpfung mit dem Filamentgarn der nächsten Spule und zur Kontrolle/Ermöglichung der optionalen Zwischenlagerzeiten, beispielsweise in einem Speicher (3) zwischen den einzelnen, darauffolgenden weiteren Schritten), wird das Filamentgarn geschnitten, so dass die Spule verpackt, gelagert und zur Weiterverwendung bereitgestellt werden kann.
4.) Das gewebte Probenstück wird nun der Weißgradmessung (4) zugeführt und anschließend kann erneut eine optionale Zwischenlagerung in einem weiteren Speicher (5) erfolgen.
5.) Anschließend erfolgt die Anfärbung des Probenstücks, beispielsweise in einem Färbefoulard (6), gefolgt von einer Dampfbehandlung (7) zur Fixierung der Anfärbung, einer Waschstufe (8) und einer Trocknung (9). Optional kann dann vor der anschließenden Farbwertbestimmung (11) eine erneute Zwischenlagerung in einem Speicher (10) erfolgen.
6.) Nach dem durchlaufen der vorstehend beschriebenen Schritte können die Probestücke durch einen Wickler (12) aufgewickelt und dann, beispielsweise in Form einer Rolle gelagert werden.

In dem erfindungsgemäßen Verfahren werden bevorzugt die relevanten Daten, also insbesondere Spulennummer (Produktinformation zum Filament bzw. Filamentgarn, wie Titer, etc.), Webparameter (Schussanzahl aber auch Art und Titer des Kettfadens), Ergebnis der Weißgradmessung, Parameter der Anfärbung, Ergebnis der Farbwertbestimmung Tag der Herstellung des Probenstücks usw. durch geeignete Verfahren automatisch gespeichert, so dass für jede evaluierte Spule, ein Datensatz mit den relevanten Informationen zur Verfügung steht. Dies kann durch automatische Erfassung der Betriebsparameter der Webmaschine, des Anfärbefoulards etc. sowie durch korrespondierende Erfassung der Messergebnisse erfolgen. Die dazu notwendigen Systeme sind dem Fachmann bekannt. Diese Daten können dann auch ganz oder teilweise in der Form eines Labels am Probenstück selbst fixiert werden, so dass auch eine direkte Identifikation der Probestücke möglich ist. Label könne in geeigneter Weise mit dem Probestück verbunden werden (kleben, anheften etc.) oder direkt, beispielsweise als Strichcode etc. auf das Probestück gedruckt werden. Die dazu notwendigen Vorrichtungen etc. sind dem Fachmann belannt.

Dadurch wird ein kompletter Datensatz an relevanten Information für jede Spule erzeugt, die über die eindeutige Identifikation des Probenstücks und der Spule eine Speicherung der relevanten Produktparameter ermöglicht.

Erfindungsgemäß wird neben dem vorstehend beschriebenen Verfahren auch eine Vorrichtung zur kontinuierlichen und vorzugsweise automatisierten Herstellung und Evaluierung von Garnen/Filamentgarnen bereitgestellt. Diese Vorrichtung umfass zumindest eine Webmaschine zur Herstellung eines gewebten Probenstücks sowie mindesten eine Einheit zur Weißgradbestimmung des Probenstücks oder eine Einheit zur Anfärbung und Farbwertbestimmung des Probestücks. Vorzugsweise sind diese Vorrichtungen durch geeignete Einrichtungen verbunden, die eine kontinuierliche und weitesgehende automatisierte Führung einer Mehrzahl an optional miteinander verbundenen Probestücken durch die Vorrichtung ermöglichen.

Unter Verweis auf Figur 1 kann eine derartige Vorrichtung also die folgenden Bestandteile aufweisen:
A.) Einen automatischen Garnzuführer (1). Dieser führt den Anfang eines Filamentgarns einer Spule in eine Webmaschine (2) ein wo dieser Anfang optional automatisch mit dem Ende des Filamentgarns der vorherigen Spule verknüpft wird. Alternative Ausgestaltungen sind, wie in Absatz [0012] beschrieben möglich. Dazu wird dann beispielsweise ein Einlegeautomat eingesetzt.
B.) Nach der Webmaschine kann ein Speicher (3) vorgesehen werden. Anschließend ist ein Weißgradmesser (4) vorgesehen, gefolgt erneut von einem optionalen Speicher (5).
C.) Anschließend kann ein Färbefoulard (6) vorgesehen werden, gefolgt von einem Dämpfer (7) zur Fixierung der Anfärbung, ein Wäscher (8) und ein Trockner (9). Optional kann dann vor der anschließenden Einheit zur Farbwertbestimmung (11) ein erneuter Speicher (10) vorgesehen sein.
D.) Zum Abschluss kann eine Einheit zur Herstellung einer kompakten Lagerungsform einer Vielzahl an Probestücken vorgesehen sein, wie ein Wickler (12), der die Probestücke zu einer gut lagerbaren Rolle an Rückhaltmustern aufrollt.

Nicht gezeigt in Figur 1 ist die für eine kontinuierliche und automatisierte Verfahrensführung notwendige Einreichung zum kontinuierlichen und gesteuerten Führen der Probestücke durch die Vorrichtung. Derartige Einrichtungen sind aber insbesondere dem Fachmann im Bereich der Filamentgarnherstellung und der Webmaschinen bekannt, beispielsweise Walzen- und Rollensysteme.

Durch das erfindungsgemäße Verfahren sowie die erfindungsgemäße Vorrichtung können also Garn-/Filamentgarnproben kontinuierlich und automatisiert zu Probestücken verarbeitet und anschließend einer Weißgradmessung und/oder Farbwertbestimmung nach einer Anfärbung unterworfen werden, so dass relevante Garn/Filamentgarndaten auf reproduzierbare Art und Weise erhalten werden können. Der Platzbedarf der Vorrichtung ist im Verglich mit Verfahren, die mit gestrickten Probestücken arbeiten geringer und auch der Durchsatz an Spulen ist erfindungsgemäß deutlich höher (kein Austausch der Strickköpfe, das System insgesamt ist aufgrund des Einsatzes einer Webmaschine robuster und weniger störungsanfällig). Die durch das erfindungsgemäße Verfahren gewonnenen Daten sind im Hinblick auf die zu vermessenden/evaluierenden Garne/Filamentgarne mit geringeren Fehlern behaftet, da die gewebten Probestücke weniger dehnbar sind, der Titer des Garns/Filamentgarns aufgrund des Einsatzes eines gewebten Probestücks keinen Einfluss auf die Bestimmungen des Weißgrads und des Farbwerts hat. Auch sind die Platzanforderungen an die Lagerung der Rückstellmuster geringer, so dass auch hier Kosten eingespart werden können. Ebenso kann durch die automatische und kontinuierliche Erfassung der relevanten Daten die Streuung der Ergebnisse, die bei manueller und batchweise durchgeführter Messung auftritt, wesentlich gemindert werden.

Durch die kontinuierliche Anfärbung der gewebten Probestücke kann drüber hinaus der Materialeinsatz und der Energieverbrauch in diesen Schritten im Verglich zu diskontinuierlichen Verfahren, insbesondere mit gestrickten Probestücken deutlich verringert werden.

Insgesamt wird also ein deutlich verbessertes System zur Erfassung relevanter Daten für Garne/Filamentgarne, insbesondere Lyocell-Filamentgarne bereitgestellt.

## Patentansprüche

1. Verfahren zur Bestimmung des Weißgrads und/oder des Farbwerts eines cellulosischen Garns/Filamentgarns, insbesondere eines Lyocell-Filamentgarns, **dadurch gekennzeichnet, dass** aus dem zu evaluierenden Garn/Filamentgarn ein gewebtes Probestück hergestellt wird, wobei das zu evaluierende Garn/Filamentgarn als Schussfaden eingesetzt wird, und wobei der Weißgrad und/oder der Farbwert des Garns/Filamentgarns, nach Anfärbung des gewebten Probestücks, auf der dominierenden schussbetonten Seite des gewebten Probestücks bestimmt wird.

2. Verfahren nach Anspruch 1, wobei als Kettfaden ein Lyocell-Filamentgarn oder eine Synthesefaser mit einem Titer von 60 bis 160 dtex verwendet wird.

3. Verfahren nach einem der Ansprüche 1 oder 2, wobei nach der Herstellung des gewebten Probestücks zunächst die Weißgradbestimmung und anschließend, nach Anfärbung des Probestücks, die Farbwertbestimmung erfolgt.

4. Verfahren nach einem der Ansprüche 1 bis 3, wobei unterschiedliche Garne/Filamentgarne nacheinander in einer kontinuierlichen Verfahrensführung zu gewebten Probestücken verarbeitet und diese kontinuierlich der Bestimmung des Weißgrads und der Bestimmung des Farbwerts, nach Anfärbung der Probestücke, unterworfen werden.

5. Verfahren nach Anspruch 4, wobei die einzelnen Probestücke miteinander durch nichtgewebte Kettabschnitte verbunden sind.

6. Verfahren nach Anspruch 4 oder 5, wobei die Probenstücke nach der Bestimmung des Weißgrads und des Farbwerts zu einer Rolle aufgewickelt werden.

7. Verfahren nach einem der Ansprüche 1 bis 6, wobei die Anfärbung durch Einsatz eines Direktfarbstoffes erfolgt.

8. Vorrichtung zur Bestimmung des Weißgrads und/oder des Farbwerts eines Garns/Filamentgarns, insbesondere eines Lyocell-Filamentgarns, umfassend eine Webmaschine, in der zu evaluierende Garne/Filamentgarne zu gewebten Probestücken verarbeitet werden, sowie eine Einheit zur Bestimmung des Weißgrads und/oder eine Einheit zur Bestimmung des Farbwerts des Garns/Filamentgarns anhand des gewebten Probestücks.

9. Vorrichtung nach Anspruch 8, umfassend einen automatischen Garnzuführer, zur Einführung eines Garns/Filamentgarns in die Webmaschine.

10. Vorrichtung nach Anspruch 8 oder 9, wobei die Einheit zur Bestimmung des Farbwerts zusätzlich ein Färbefoulard, eine Dampfeinheit zur Fixierung, einen Wäscher und einen Trockner umfasst.

11. Vorrichtung nach einem der Ansprüche 8 bis 10, weiter umfassend eine Einheit zum kontinuierlichen Führen einer Vielzahl and Probestücken durch die Vorrichtung.

12. Vorrichtung nach einem der Ansprüche 8 bis 11, weiter umfassend Systeme zur automatisierten Erfassung der Daten der Webmaschine, der Einheit zur Weißgradmessung sowie der Einheit zur Bestimmung des Farbwerts.
